# EUROPEAN PATENT APPLICATION

(11) **EP 4 286 052 A1**
(43) Date of publication of application: **06.12.2023**
(21) Application number: 22746148.0
(22) Date of filing: 19.01.2022
(51) Int. Cl.: B01J 37/16, B01J 37/02, B01J 37/08, B01J 23/62, B01J 23/656, C07C 29/149, C07C 35/14

(54) **METHOD FOR MANUFACTURING HETEROGENEOUS METAL HYDROGENATION CATALYST**

(30) Priority: 01.02.2021 KR 20210013970
(71) Applicant: Hanwha Solutions Corporation, Jung-gu Seoul 04541 (KR)
(72) Inventor: KIM, Jeong Kwon, Daejeon 34128 (KR); JEON, Bong Sik, Daejeon 34128 (KR)
(74) Representative: Berggren Oy
(86) International application number: PCT/KR2022/000997
(87) International publication number: WO 2022/164121

(57) **Abstract**

The present invention relates to a method for manufacturing a heterogeneous metal hydrogenation catalyst. More specifically, the present invention is characterized in that when the hydrogenation catalyst is reduced using a specific reducing gas in a proper reducing condition, the hydrogenation reaction of the catalyst is improved.

## Description

### [Technical Field]

The present invention relates to a method of preparing a bimetallic hydrogenation catalyst and, more particularly to, a method of preparing a bimetallic hydrogenation catalyst having improved catalytic activity in hydrogenation through reduction of the catalyst under appropriate reduction conditions using a specific reducing gas.

### [Background Art]

A heterogeneous hydrogenation catalyst may be prepared by supporting, drying, reducing, and passivating an active metal. In order to have catalytic activity, the catalyst is activated through a process in which an active metal in the form of a supported metal compound is reduced to a metal. Catalyst activation may be achieved by reducing the active metal prior to reaction through slurry reduction and thermal reduction.

Slurry reduction has an advantage of using a reducing agent with high hydrogen content, such as hydrazine hydrate (N₂H₄·H₂O) and sodium borohydride (NaBH₄), to reduce an active metal to a metal in a short time at a relatively low temperature. As a catalyst reducing agent, although hydrazine hydrate (N₂H₄·H₂O) is highly effective in catalyst reduction in small amounts due to a high reduction potential thereof, hydrazine hydrate can cause respiratory toxicity and environmental pollution. Chinese Patent Registration No. 102580732 (Beijing University of Chemical Technology) discloses Ru-Pt-Sn/Al₂O₃ as a catalyst for conversion of dicarboxylic acid, wherein a slurry reduction method using NaBH₄ is employed to reduce the catalyst. NaBH₄, which has high hydrogen content, is hydrolyzed in an aqueous solution to generate hydrogen, thereby reducing a metal compound. However, Na, B, and the like generated upon hydrolysis of NaBH₄ can act as catalyst poisons due to strong binding ability to metals, and thus can reduce catalytic activity.

Thermal reduction is a method of reducing a metal salt to a metal by supplying a reducing agent to a catalyst precursor under heat, wherein the reducing agent includes hydrogen, carbon dioxide, carbon monoxide, methane, and the like, specifically hydrogen. Concentrated hydrogen mixed with air in a certain mixing ratio is prone to deflagration or explosion. Accordingly, a mixture of hydrogen and an inert gas (about 2% to 10% hydrogen) is commonly used in commercial processes to secure safety.

In order to apply thermal reduction to a bimetallic catalyst, appropriate reduction conditions are required due to different thermodynamic properties between two metals constituting the bimetallic catalyst. Related literature has reported that the degree of reduction of a bimetallic catalyst depends on the reduction temperature and the composition of a reducing gas and, when two metals constituting the bimetallic catalyst form an alloy, a ratio between the two metals in an alloy phase varies depending on reduction conditions employed, which influences catalytic performance of the catalyst (Appl. Catal. A-Gen 315 (2006) 58-67).

After years of research to solve these problems, the inventors completed the present invention based on confirmation that a bimetallic hydrogenation catalyst reduced under appropriate reduction conditions using a specific reducing gas can be used to efficiently convert a dicarboxylic acid group to a dialcohol group.

### [Disclosure]

### [Technical Problem]

The present invention is conceived to solve such problems in the art and it is one object of the present invention to provide a method of reducing a bimetallic hydrogenation catalyst.

It is another object of the present invention to provide a method of preparing cyclohexane dimethanol (CHDM) using a bimetallic hydrogenation catalyst activated by the method set forth above.

### [Technical Solution]

In accordance with one aspect of the present invention,
a method of reducing a bimetallic hydrogenation catalyst includes: charging a reactor with a catalyst precursor including a bimetallic compound supported on a carrier; and reducing the bimetallic compound in the catalyst precursor by heating the reactor while supplying a reducing gas to the reactor.

The bimetallic compound may include a first metal and a second metal, wherein the first metal may include a compound of a metal selected from the group consisting of Ru, Pt, Pd, Rh, and combinations thereof and the second metal may include a compound of a metal selected from the group consisting of Sn, Fe, Ga, Re, and combinations thereof.

A molar ratio of the first metal to the second metal may range from 1:0.5 to 1:3.

The carrier may include a material selected from the group consisting of silica (SiO₂), alumina (Al₂O₃), zirconia (Zr0₂), titania (TiO₂), carbon, and combinations thereof.

The carbon may include a material selected from the group consisting of activated carbon, carbon black, graphite, graphene, ordered mesoporous carbon (OMC), carbon nanotubes (CNTs), and combinations thereof.

The bimetallic compound may be present in an amount of 1 part by weight to 20 parts by weight based on 100 parts by weight of the carrier.

The reactor may be heated at a rate of 1°C/min to 15°C /min.

Reduction of the bimetallic compound may be carried out in a kiln, a furnace, or a reactor with a fixed bed type, a fluidized bed type, a moving bed type or a static box type.

The reducing gas may include a gas selected from the group consisting of hydrogen (H₂), carbon monoxide (CO), carbon dioxide (CO₂), methane (CH₄), ammonia (NH₃), hydrogen sulfide (H₂S), and combinations thereof.

The reducing gas may be supplied in an amount greater than or equal to the number of moles of the bimetallic compound per unit mass of the catalyst precursor during heating of the reactor.

The method may further include: subsequent to reducing the bimetallic compound by heating the reactor, maintaining the temperature of the reactor.

The reactor may be maintained at a temperature of 200°C to 500°C.

Heating and temperature maintenance of the reactor may be performed for 30 minutes to 24 hours.

The bimetallic catalyst may be used in hydrogenation.

Hydrogenation using the bimetallic catalyst may reduce a carboxylic acid group, an aldehyde group, or a ketone group to an alcohol group.

Hydrogenation using the bimetallic catalyst may reduce a dicarboxylic acid group to a dialcohol group.

In accordance with another aspect of the present invention,
there is provided a method of preparing cyclohexane dimethanol (CHDM) through hydrogenation of cyclohexanedicarboxylic acid (CHDA) using the bimetallic hydrogenation catalyst activated by the method set forth above.

Cyclohexane dimethanol (CHDM) may be prepared at a yield of 70% or more.

### [Advantageous Effects]

The method of reducing a bimetallic hydrogenation catalyst according to the present invention may control segregation, sintering, and leaching out of an active metal, which can occur during a process of reducing of a bimetallic compound to a metal, and may obtain a bimetallic hydrogenation catalyst including a supported active metal having a homogeneous alloy phase.

In addition, the bimetallic hydrogenation catalyst reduced by the method set forth above may efficiently reduce a dicarboxylic acid group to a dialcohol group.

### [Description of Drawings]

FIG. 1 is an image showing results of STEM-EDX analysis of bimetallic catalysts according to Examples of the present invention.
FIG. 2 is an image showing results of STEM-EDX analysis of bimetallic catalysts according to Comparative Examples of the present invention.

### [Mode for Invention]

Hereinafter, exemplary embodiments of the present invention will be described with reference to the accompanying drawings. It should be understood that the present invention may be embodied in different ways and is not limited to the following embodiments and that the scope of the present invention is only defined by the appended claims.

The terminology used herein is for the purpose of describing particular embodiments and is not intended to be limiting. As used herein, the singular forms, "a," "an," and "the", are intended to include the plural forms as well, unless the context clearly indicates otherwise. In addition, the terms "comprises," "comprising," "includes," and/or "including," when used in this specification, specify the presence of stated features, integers, steps, operations, elements, components, and/or groups thereof, but do not preclude the presence or addition of one or more other features, integers, steps, operations, elements, components, and/or groups thereof.

A first aspect of the present invention provides
a method of reducing a bimetallic hydrogenation catalyst, including: charging a reactor with a catalyst precursor including a bimetallic compound supported on a carrier; and reducing the bimetallic compound in the catalyst precursor to a metal by heating the reactor while supplying a reducing gas to the reactor.

Catalytic activity of bimetallic hydrogenation catalysts strongly correlates with the morphology, ratio, and dispersion of active metals, depending on their intended use (Appl. Catal. A-Gen. 318 (2007) 70-78). A bimetallic hydrogenation catalyst is a bifunctional catalyst in which the roles of the heterogeneous active metals are different and is effective in promoting hydrogenation of a carboxylic acid group or a carbonyl group by electronic ensemble effects induced by alloying a metal having high oxygen affinity (for example, Sn, Re, Ga, Fe, and the like) with a noble metal. Therefore, in order to obtain a bimetallic hydrogenation catalyst having good catalytic activity, it is necessary to design appropriate active metals (Appl. Catal. A-Gen. 318 (2007) 70-78).

In order for a supported catalyst precursor to have catalytic activity in hydrogenation, a process of reducing the bimetallic compound to its metallic form is necessary. Such a supported catalyst reduction process generally employs a thermal reduction method in which a supported catalyst is heated to a target reduction temperature while supplying a mixed gas comprising hydrogen. Here, insufficient reduction conditions during the heating process make it impossible to obtain catalytic properties and performance as designed. That is, an excessively high heating rate or an excessively high target reduction temperature can cause metal particles to migrate or agglomerate with each other on the surface of the catalyst during reduction, resulting in grain growth (sintering) and thus reduction in effective reaction surface area of active sites participating in reaction. Thus, appropriate reduction conditions for each metal species are essential (J. A. Anderson et al. "Supported Metals in Catalysis", Imperial college press).

Accordingly, the present invention is aimed at solving the aforementioned problems through reduction of a bimetallic hydrogenation catalyst under appropriate reduction conditions using a specific reducing gas.

Now, each step of the method of reducing a bimetallic hydrogenation catalyst according to the first aspect of the present invention will be described in detail.

In one embodiment, the method of reducing a bimetallic hydrogenation catalyst may include: charging a reactor with a catalyst precursor including a bimetallic compound supported on a carrier.

In one embodiment, the bimetallic compound may include a first metal and a second metal, wherein the first metal may include a compound of a metal selected from the group consisting of Ru, Pt, Pd, Rh, and combinations thereof, and the second metal may include a compound of a metal selected from the group consisting of Sn, Fe, Ga, Re, and combinations thereof. Here, a molar ratio of the first metal to the second metal may be 1:0.5 to 1:3, preferably 1:1. More preferably, the first metal and the second metal may be present in a molar ratio of 1:1 after reduction. If the second metal is present in an amount of less than 0.5 moles per 1 mole of the first metal, it may inhibit the activation of a carboxylic acid group, resulting in low selectivity of desired products. Conversely, if the second metal is present in an amount of greater than 3 moles per 1 mole of the first metal, it may inhibit generation of a metal-hydride compound involved in hydrogenation, rendering the catalyst unsuitable for use in promoting hydrogenation.

In one embodiment, the first metal and the second metal are used as an active metal, and may have a metal crystallite size of 1 nm to 20 nm, preferably 1 nm to 15 nm. If the first metal and the second metal have a metal crystallite size exceeding 20 nm, this makes it difficult to obtain a high catalytic conversion rate.

In one embodiment, the carrier may include a material selected from the group consisting of silica (SiO₂), alumina (Al₂O₃), zirconia (ZrO₂), titania (TiO₂), carbon, and combinations thereof. Preferably, the carrier may include carbon. Here, the carbon may include a material selected from the group consisting of activated carbon, carbon black, graphite, graphene, ordered mesoporous carbon (OMC), carbon nanotubes (CNTs), and combinations thereof.

In one embodiment, when the carrier is a carbon carrier, mesopores of the carbon carrier, ranging in size from 2 nm to 50 nm, may occupy 50% or more of the total pore volume of the carrier. Preferably, the mesopores of the carbon carrier may occupy 70% or more of the total pore volume of the carrier, more preferably 75% or more of the total pore volume of the carrier. If the volume fraction of the mesopores is less than 50%, this can slow down microscopic mass transfer of a reactant and a product in the carbon carrier and, if the carbon carrier has an average pore size of greater than 50 nm, the carbon carrier can have poor physical strength. Accordingly, the above range of volume fraction of the mesopores is considered appropriate.

In one embodiment, the carbon may include activated carbon having a specific surface area (BET) of 100 m²/g to 1,500 m²/g. Preferably, the carbon may include activated carbon having a specific surface area (BET) of 200 m²/g to 1,000 m²/g. If the specific surface area of the carbon is less than 100 m²/g, this cannot ensure high dispersion of the active metal on the carbon, whereas, if the specific surface area of the carbon exceeds 1,500 m²/g, this can result in decrease in volume fraction of mesopores. Accordingly, the above range of specific surface area of the carbon is considered appropriate.

In one embodiment, the carbon carrier may include an appropriate fraction of micropores in addition to mesopores of medium size. Preferably, the micropores may occupy 25% or less of the total pore volume of the carbon carrier. If the volume fraction of the micropores exceeds 25%, this can slow down microscopic mass transfer of a reactant and a product in the carbon carrier. In this sense, the above range of volume fraction of the micropores is considered appropriate.

In one embodiment, the bimetallic compound may be present in an amount of 1 part by weight to 20 parts by weight based on 100 parts by weight of the carrier, specifically 1 part by weight to 10 parts by weight based on 100 parts by weight of the carrier, preferably 3 parts by weight to 7 parts by weight based on 100 parts of the carrier. If the content of the bimetallic compound is less than 1 part by weight based on 100 parts by weight of the carrier, this can cause low catalytic conversion efficiency or low selectivity of desired products, resulting in excessive separation and recovery costs in related processes, whereas, if the content of the bimetallic compound exceeds 20 parts by weight based on 100 parts of the carrier, this results in high yield of undesirable by-products, which is economically inefficient.

In one embodiment, the hydrogenation catalyst may have an average particle size (d₅₀) of 3 µm to 50 µm. If the average particle size of the catalyst is less than this range, loss of the catalyst can occur during passage of the catalyst through a filtration membrane, resulting in reduction in purity of a product and additional cost due to loss of the catalyst, whereas, if the average particle size of the catalyst exceeds this range, this can lead to poor catalytic efficiency in hydrogenation due to low dispersion of the catalyst in a reaction medium.

In one embodiment, the active metal of the hydrogenation catalyst may have a homogenous miscible phase. When the active metal includes, for example, ruthenium (Ru) and tin (Sn), Ru and Sn may form a homogenous miscible phase rather than existing independently of each other.

In this regard, it has been reported in literature that catalytic activity of a bimetallic hydrogenation catalyst correlates with homogenous composition of an active metal thereof (J.Mol. Catal A Chem 2015, 410, 184). Therefore, achieving a high degree of homogeneity between two metals that form the active metals is crucial to obtain good catalytic activity for a given amount of supported metal. In the bimetallic hydrogenation catalyst, the first metal serves to produce a metal-hydride compound through adsorption of hydrogen, while the second metal acts as a Lewis acid to polarize a carbonyl group. Since the metal-hydride compound is then converted into alcohol through adsorption onto an activated carbonyl group, a homogenous bimetallic active phase is essential for efficient reduction of the carbonyl group. Thus, the catalyst according to the present invention contains a homogenous bimetallic active phase, thereby improving catalytic efficiency in hydrogenation.

In one embodiment, the method of reducing a bimetallic hydrogenation catalyst may include: reducing the bimetallic compound in the catalyst to a metal by heating the reactor while supplying a reducing gas to the reactor.

In one embodiment, the reducing gas may include a gas selected from the group consisting of hydrogen (H₂), carbon monoxide (CO), carbon dioxide (CO₂), methane (CH₄), ammonia (NH₃), hydrogen sulfide (H₂S), and combinations thereof. Preferably, the reducing gas may include hydrogen (H₂), which has a high reduction potential.

In one embodiment, the reactor may be heated at a rate of 1°C/min to 15°C/min, preferably at a rate of 1°C/min to 5°C/min. If the reactor is heated at a rate of less than 1°C/min, this is inefficient since it takes a long time for the reactor to reach a target temperature, whereas, if the reactor is heated at a rate exceeding 15°C/min, this can cause accelerated metal sintering due to rapid supply of a heat source under insufficient reducing conditions.

In one embodiment, reduction of the bimetallic compound is preferably carried out in a fixed bed type, a fluidized bed type, a moving bed type or a static box type of a kiln, a furnace or a reactor so as to ensure easy introduction and discharge of the reducing gas and to allow supply of the reducing gas to be simultaneously performed with heating of the reactor.

In one embodiment, during heating of the reactor, the reducing gas may be supplied in an amount greater than or equal to the number of moles of the bimetallic compound per unit mass of the bimetallic hydrogenation catalyst. If the amount of the reducing gas supplied is less than the number of moles of the bimetallic compound per unit mass of the bimetallic hydrogenation catalyst or if there is no supply of the reducing gas, the two metals can undergo segregation, rather than forming an alloy, and can leach out of the catalyst during heat treatment of the reactor, making it impossible to obtain a catalyst having desired properties as designed. In addition, such insufficient reducing conditions cannot ensure effective removal of ligand from the bimetallic compound, causing reduction in reaction efficiency and generation of side reactions.

In one embodiment, the method of reducing a bimetallic hydrogenation catalyst may further include: subsequent to reducing the bimetallic compound by heating the reactor, maintaining the temperature of the reactor.

In one embodiment, the reactor may be maintained at a temperature of 200°C to 500°C, preferably 350°C to 450°C. Here, the step of maintaining the temperature of the reactor after heating the reactor serves to complete reduction of the bimetallic hydrogenation catalyst. If the reactor is maintained at a temperature of less than 200°C, this cause incomplete reduction of the bimetallic compound to a metal and generation of side reactions due to insufficient removal of ligand from the bimetallic compound, whereas, if the reactor is maintained at a temperature exceeding 500°C, this can cause accelerated metal sintering.

In one embodiment, heating and temperature maintenance of the reactor may be carried out for 30 minutes to 24 hours. If heating and temperature maintenance of the reactor is carried out for less than 30 minutes, this is insufficient to ensure smooth reduction of the bimetallic hydrogenation catalyst, whereas, if heating and temperature maintenance of the reactor is carried out for more than 24 hours, this is economically disadvantageous since there is no further improvement in reduction of the bimetallic hydrogenation catalyst.

In one embodiment, the bimetallic hydrogenation catalyst may be used in hydrogenation. Here, hydrogenation using the bimetallic hydrogenation catalyst may reduce a carboxylic acid group, an aldehyde group, or a ketone group to an alcohol group, preferably to reduce a dicarboxylic acid group to a dialcohol group, more preferably, to reduce cyclohexanedicarboxylic acid (CHDA) to cyclohexane dimethanol (CHDM).

In one embodiment, carboxylic acids having the carboxylic acid group may include, for example, a material selected from the group consisting of oxalic acid, malonic acid, succinic acid, glutaric acid, adipic acid, pimelic acid, suberic acid, azelaic acid, sebacic acid, phthalic acid, isophthalic acid, terephthalic acid, formic acid, acetic acid, caproic acid, caprylic acid, lauric acid, myristic acid, palmitic acid, stearic acid, isostearyl acid, oleic acid, maleic acid, adipic acid, sebacic acid, cyclohexanecarboxylic acid, benzoic acid, and combinations thereof.

In one embodiment, aldehydes having the aldehyde group may include, for example, a material selected from the group consisting of formaldehyde, propionaldehyde, n-butyraldehyde, isobutyraldehyde, valeraldehyde, 2-methylbutyraldehyde, 3-methylbutyraldehyde, 2,2-dimethylpropionaldehyde, capronaldehyde, 2-methylvaleraldehyde, 3-methylvaleraldehyde, 4-methylvaleraldehyde, 2-ethylbutyraldehyde, 2,2-dimethylbutyraldehyde, 3,3-dimethylbutyraldehyde, caprylaldehyde, caprinaldehyde, glutardialdehyde, and combinations thereof.

In one embodiment, ketones having the ketone group may include, for example, a material selected from the group consisting of acetone, butanone, pentanone, hexanone, cyclohexanone, acetophenone, and combinations thereof.

A second aspect of the present invention provides
a method of preparing clohexane dimethanol (CHDM) through hydrogenation of cyclohexanedicarboxylic acid (CHDA) using the bimetallic hydrogenation catalyst reduced by the method according to the first aspect of the present invention.

Although common features, functions, and elements shared by the first aspect of the present invention will not be redundantly described herein, description given as to the first aspect can be applied equally to the second aspect.

In the following, the method of preparing cyclohexane dimethanol (CHDM) according to the second aspect of the present invention will be described in detail.

In one embodiment of the present disclosure, hydrogenation of CHDA may be carried out at a temperature of 200°C to 300°C and a pressure of 50 bar to 150 bar for 2 to 24 hours. Preferably, hydrogenation of CHDA may be carried out at a temperature of 200°C to 270°C and a pressure of 70 bar to 130 bar. If hydrogenation of CHDA is carried out at a temperature of less than 180°C, this results in an insufficient reaction rate, making it impossible to achieve a desired CHDM yield, whereas, if hydrogenation of CHDA is carried out at a temperature exceeding 300°C, this can cause side reactions such as decomposition of a reactant and a product. In addition, if hydrogenation of CHDA is carried out at a pressure less than 50 bar, this can slow down a rate of hydrogenation of CHDA since there is no sufficient hydrogen participating in hydrogenation in a solvent, whereas, if hydrogenation of CHDA is carried out at a pressure exceeding 150 bar, this is economically disadvantageous since there is no further improvement in reaction rate. More preferably, hydrogenation of CHDA may be carried out at a temperature of 200°C to 250°C and a pressure of 80 bar to 110 bar for 2 to 6 hours.

In one embodiment, cyclohexane dimethanol (CHDM) may be prepared at a yield of at least 70%, preferably at least 85%, more preferably at least 95%.

Next, the present invention will be described in more detail with reference to some examples. However, it should be noted that these examples are provided for illustration only and are not to be construed in any way as limiting the present invention.

### Preparative Example: Preparation of catalyst precursor

A catalyst precursor was prepared by supporting metallic Ru and Sn on a carbon carrier through incipient-wetness impregnation. Specifically, a ruthenium compound (RuCl₃·3H₂O) and a tin compound (SnCl₂·2H₂O) were weighed and dissolved in ultrapure water and then the carbon carrier was co-impregnated with the resulting solution by stirring in a mortar with a pestle while introducing the solution onto the carbon carrier dropwise. Among the two metals forming a bimetallic active phase, Ru was supported in an amount of 5 parts by weight based on 100 parts by weight of the carbon carrier and Sn was supported in an amount of equal to the number of moles of Ru. Thereafter, the supported specimen was dried in a drying oven at 100°C for 12 hours, thereby preparing a bimetallic catalyst including an Ru-Sn bimetallic compound supported on carbon.

### Example 1: Reduction of catalyst precursor (using 5% H₂)

Reduction of the catalyst precursor prepared in Preparative Example was performed in a fixed bed reactor. Here, equipment for reduction of the catalyst precursor included a reactor adapted to be charged with a catalyst, a reactor heater to control the temperature of the specimen, and a mass flow controller (MFC) to control the flow rate of gas. Reduction of the catalyst precursor was performed by heat treatment in which the specimen was programmatically heated to a target reduction temperature using a PID controller while supplying a reducing gas to the reactor. Here, the reducing gas was mixed hydrogen gas (5% hydrogen, balance N₂) and the specimen was heated to 350°C (the target reduction temperature) at a rate of 5°C/min, followed by maintaining the temperature of the reactor at 350°C for 3 hours. During the heating step of the reduction process, hydrogen was supplied to the reactor at a controlled flow rate per unit mass of the catalyst precursor (F/W: H₂-ml/min.g-Cat.), and the total amount of hydrogen input is shown in Table 1. A catalyst thus reduced was cooled to room temperature by supplying nitrogen (F/W: 20 ml-N₂/min.g-Cat.) to the reactor and then was passivated by supplying 3% oxygen-nitrogen mixed gas (F/W: 20 ml/min.g-Cat.) to the reactor at room temperature for 2 hours.

### Example 2: Reduction of catalyst precursor (using 100% H₂)

Reduction of the catalyst precursor was performed in the same manner as in Example 1 except that pure hydrogen gas (100% hydrogen) was used as the reducing gas instead of mixed hydrogen gas (5% hydrogen).

### Example 3. Reduction of catalyst precursor (using 100% H₂ only in the heating step)

Reduction of the catalyst precursor was performed in the same manner as in Example 1 except that pure hydrogen gas (100% hydrogen) was used as the reducing gas instead of mixed hydrogen gas (5% hydrogen), wherein the hydrogen gas was only supplied during the heating step.

Specifically, reduction of the catalyst precursor prepared in Preparative Example was performed by heating the reactor to 350°C at a rate of 5°C/min and maintaining the temperature of the reactor for 3 hours. Pure hydrogen was only supplied during the heating step (for about 70 minutes) of the reduction process and was replaced with nitrogen gas during the temperature maintenance step. A catalyst thus reduced was cooled to room temperature by supplying nitrogen (F/W: 20 ml-N₂/min.g-Cat.) to the reactor and then was passivated by supplying 3% oxygen-nitrogen mixed gas (F/W: 20 ml/min.g-Cat.) to the reactor at room temperature for 2 hours.

### Comparative Example 1: Reduction of catalyst precursor (using 100% N₂)

Reduction of the catalyst precursor was performed in the same manner as in Example 1 except that pure nitrogen (N₂) gas (100% nitrogen) was used as the reducing gas instead of mixed hydrogen gas (5% hydrogen)

### Comparative Example 2: Reduction of catalyst precursor (using 100% N₂/100% H₂)

Reduction of the catalyst precursor prepared in Preparative Example was performed by heating the reactor to 350°C at a rate of 5°C/min and maintaining the temperature of the reactor at 350°C for 3 hours. Here, nitrogen gas was supplied during the heating step of the reduction process and, after the temperature of the reactor reached 350°C, hydrogen gas was supplied for 180 min in place of the nitrogen gas. A catalyst thus reduced was cooled to room temperature by supplying nitrogen (F/W: 0.05 g-Cat.min/ml) to the reactor and then was passivated by supplying 3% oxygen-nitrogen mixed gas (F/W: 0.05 g-Cat.min/ml) to the reactor at room temperature for 2 hours.

### Experimental Example: Experiments on hydrogenation conversion of cyclohexanedicarboxylic acid (CHDA)

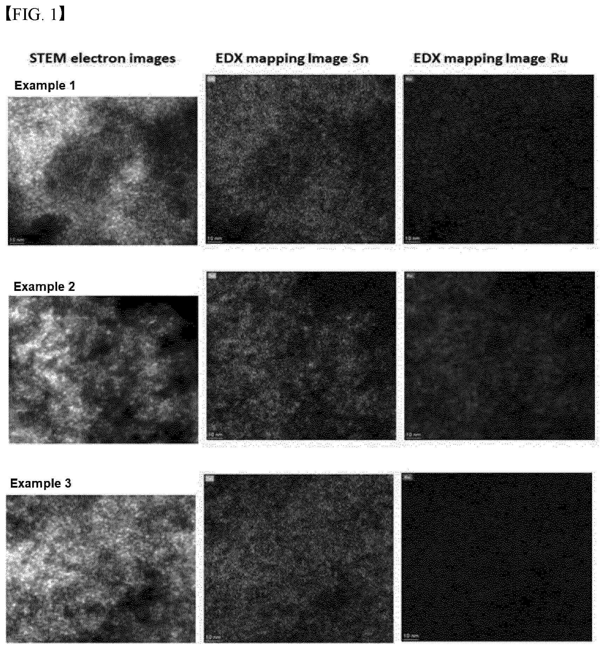

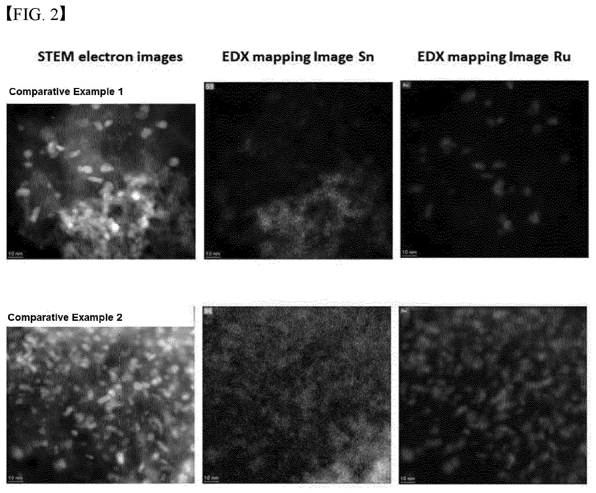

Experiments were conducted in preparation of cyclohexane dimethanol (CHDM) through hydrogenation of dicarboxylic acid, specifically cyclohexanedicarboxylic acid (CHDA). Hydrogenation of CHDA was carried out in an acid-resistant titanium-lined stainless steel batch reactor with a maximum working pressure of 100 bar. CHDA as a reactant and each of the reduced bimetallic hydrogenation catalysts prepared in Examples 1 to 3 and Comparative Examples 1 to 2 were introduced in a weight ratio of 3.75: 1 into the reactor, followed by charging the reactor with distilled water as a reaction solvent. Here, the amount of the reactant was fixed at 1.6 wt% relative to the weight of the solvent. Thereafter, hydrogen was supplied to the reactor and compressed to a reaction pressure (90 bar), followed by checking the reactor for leaks using a hydrogen detector, and then oxygen was completely removed from the reactor through depressurization and purging. Hydrogenation of the reactant was carried out by raising the internal temperature of the reactor to a reaction temperature (230°C), followed by stirring a reaction mixture at 1,000 rpm for 6 hours using an overhead stirrer under the reaction pressure (90 bar) in a hydrogen atmosphere. The resulting product was sampled over time through a metal filter, followed by silylation with N,O-Bis(trimethylsilyl)trifluoroacetamide (BSTFA), and then the product and the residual reactant were analyzed using a gas chromatograph (DS-SCIENCE Inc.) equipped with an HP-1 column (Agilent Technologies). Results are shown in FIG. 1, FIG. 2, and Table 1.

**<Table 1>**

| Item | Total F/W^{a}) (mL/min.g-Cat.) | Hydrogen F/W (mL/min.g-Cat.) | Amount of hydrogen input during heating (mmol-H2/g-Cat.) | Amount of hydrogen input during temperature maintenance (mmol-H2/g-Cat.) | Sn/Ru atomic ratio | CHDM yield (%) |
|---|---|---|---|---|---|---|
| Example 1 | 20 | 1 | 14.4 | 7.4 | 1.02 | 86 |
| Example 2 | 20 | 20 | 57.5 | 148 | 1.04 | 86 |
| Example 3 | 20 | 20 | 57.5 | 0 | 1.10 | 72 |
| Comparative Example 1 | 20 | 0 | 0 | 0 | 0.42 | 0.8 |
| Comparative Example 2 | 20 | 20 | 0 | 147.8 | 0.86 | 26 |

| | | | | | | |
|---|---|---|---|---|---|---|
| a) F/W: volumetric flow rate per catalyst precursor weight (ml/min.g-Cat.) | | | | | | |

The analysis results clearly showed differences in metal dispersion, crystallite size and composition of an active metal depending on the type of reducing gas introduced during the heating step.

Specifically, FIG. 1 shows the results of STEM-EDX (energy dispersive X-ray spectroscopy) mapping analysis of catalysts supplied with hydrogen during the heating step (Examples 1 to 3). STEM images of the catalysts showed that metal crystals had a size of less than 5 nm and active components were uniformly distributed on the carbon carrier. In addition, Table 1 shows an atomic ratio of Sn to Ru (Sn/Ru) at an active site, which was obtained from the results of EDX mapping analysis. Therefrom, it could be seen that there was little or no difference in Sn/Ru before and after reduction, indicating that the catalysts were prepared as designed.

FIG. 2 shows the results of STEM-EDX (energy dispersive x-ray spectroscopy) mapping analysis of catalysts supplied with nitrogen during the heating step (Comparative Examples 1 to 2). STEM images of the catalysts showed that metal crystals partially form agglomerates having a size of 10 nm or more and Ru was dominant in the agglomerates, indicating that phase segregation occurred during reduction. In addition, from the results of EDX mapping analysis, it could be seen that Sn/Ru after reduction was lower than that before reduction, that is, a proportion of Sn decreased after reduction, indicating that the catalysts were not prepared as designed.

Table 1 also shows the amount of hydrogen input and catalytic activity in terms of CHDM yield under different catalyst reduction conditions. The catalysts supplied with hydrogen during reduction exhibited high CHDM yields of 70% or more, whereas the catalysts supplied with nitrogen during reduction exhibited low CHDM yields. Catalytic activity of a bimetallic catalyst depends on the ratio between Sn and Ru forming the active metal (Sn/Ru) and the amount of hydrogen available to participate in reaction. In the case of the catalysts supplied with nitrogen during the heating step, supply of a heat source was performed under insufficient reduction conditions, resulting in segregation of active sites or leaching out of metallic Sn. Consequently, it was confirmed that a reducing gas supplied during the heating step greatly influences catalyst properties, particularly the effective reaction surface area of the active metal, including the size and dispersion of active metal particles, and thus is an important factor determining catalytic activity in conversion of CHDA to CHDM.

Although some embodiments have been described herein with reference to the accompanying drawings, it should be understood that these embodiments are provided for illustration only and are not to be construed in any way as limiting the present invention, and that various modifications, changes, alterations, and equivalent embodiments can be made by those skilled in the art without departing from the spirit and scope of the invention. Therefore, the scope of the present invention should be defined by the appended claims and equivalents thereto.

### [Industrial Applicability]

The method of reducing a bimetallic hydrogenation catalyst according to the present invention may control segregation, sintering, and leaching out of an active metal, which can occur during a process of reducing of a bimetallic compound to a metal and may obtain a bimetallic hydrogenation catalyst including a supported active metal having homogenous alloy phase.

In addition, the bimetallic hydrogenation catalyst reduced by the method set forth above may efficiently reduce a dicarboxylic acid group to a dialcohol group.

## Claims

1. A method of reducing a bimetallic hydrogenation catalyst, comprising:
charging a reactor with a catalyst precursor comprising a bimetallic compound supported on a carrier; and
reducing the bimetallic compound in the catalyst precursor by heating the reactor while supplying a reducing gas to the reactor.

2. The method according to claim 1, wherein the bimetallic compound comprises a first metal and a second metal,
the first metal comprising a compound of a metal selected from the group consisting of Ru, Pt, Pd, Rh, and combinations thereof,
the second metal comprising a compound of a metal selected from the group consisting of Sn, Fe, Ga, Re, and combinations thereof.

3. The method according to claim 2, wherein a molar ratio of the first metal to the second metal ranges from 1:0.5 to 1:3.

4. The method according to claim 1, wherein the carrier comprises a material selected from the group consisting of silica (SiO₂), alumina (Al₂O₃), zirconia (ZrO₂), titania (TiO₂), carbon, and combinations thereof.

5. The method according to claim 4, wherein the carbon comprises a material selected from the group consisting of activated carbon, carbon black, graphite, graphene, ordered mesoporous carbon (OMC), carbon nanotubes (CNTs), and combinations thereof.

6. The method according to claim 1, wherein the bimetallic compound is present in an amount of 1 part by weight to 20 parts by weight based on 100 parts by weight of the carrier.

7. The method according to claim 1, wherein the reactor is heated at a rate of 1°C/min to 15°C /min.

8. The method according to claim 1, wherein reduction of the bimetallic compound is carried out in a kiln, a furnace, or a reactor with a fixed bed type, a fluidized bed type, a moving bed type or a static box type.

9. The method according to claim 1, wherein the reducing gas comprises a gas selected from the group consisting of hydrogen (H₂), carbon monoxide (CO), carbon dioxide (CO₂), methane (CH₄), ammonia (NH₃), hydrogen sulfide (H₂S), and combinations thereof.

10. The method according to claim 1, wherein the reducing gas is supplied in an amount greater than or equal to the number of moles of the bimetallic compound per unit mass of the catalyst precursor during heating of the reactor.

11. The method according to claim 1, further comprising: subsequent to reducing the bimetallic compound by heating the reactor,
maintaining the temperature of the reactor.

12. The method according to claim 11, wherein the reactor is maintained at a temperature of 200°C to 500°C.

13. The method according to claim 11, wherein heating and temperature maintenance of the reactor are performed for 30 minutes to 24 hours.

14. The method according to claim 1, wherein the catalyst is used in hydrogenation.

15. The method according to claim 14, wherein hydrogenation using the catalyst reduces a carboxylic acid group, an aldehyde group, or a ketone group to an alcohol group.

16. The method according to claim 14, wherein hydrogenation using the catalyst reduces a dicarboxylic acid group to a dialcohol group.

17. A method of preparing cyclohexane dimethanol (CHDM) through hydrogenation of cyclohexanedicarboxylic acid (CHDA) using the bimetallic hydrogenation catalyst activated by the method according to claim 1.

18. The method according to claim 17, wherein cyclohexane dimethanol (CHDM) is prepared at a yield of 70% or more.
